# EUROPEAN PATENT APPLICATION

(11) **EP 4 102 510 A1**
(43) Date of publication of application: **14.12.2022**
(21) Application number: 22159683.6
(22) Date of filing: 02.03.2022
(51) Int. Cl.: G16C 20/40

(54) **STABLE STRUCTURE SEARCH SYSTEM, STABLE STRUCTURE SEARCH METHOD, AND STABLE STRUCTURE SEARCH PROGRAM**

(30) Priority: 09.06.2021 JP 2021096771
(71) Applicant: FUJITSU LIMITED, Kawasaki-shi, Kanagawa 211-8588 (JP)
(72) Inventor: Terashima, Chieko, Kawasaki-shi, Kanagawa, 211-8588 (JP); Tanida, Yoshiaki, Kawasaki-shi, Kanagawa, 211-8588 (JP); Sato, Hiroyuki, Kawasaki-shi, Kanagawa, 211-8588 (JP)
(74) Representative: Hoffmann Eitle

(57) **Abstract**

A stable structure search system includes a calculation unit that acquires a plurality of degrees of structure similarity between each of a plurality of kinds of molecules and a first molecule included in a target molecule based on each interaction potential of the plurality of kinds of molecules, acquires a plurality of degrees of charge similarity between each of the plurality of kinds of molecules and the first molecule, and acquires a plurality of total degrees of similarity based on sums of each of the plurality of degrees of structure similarity and each of the plurality of degrees of charge similarity; and an identification unit that determines a second molecule whose total degree of similarly is largest among the plurality of kinds of molecules, acquires energy of a molecular structure of the target molecule based on an interaction potential of the second molecule.

## Description

### FIELD

The embodiments discussed herein are related to a stable structure search system, a stable structure search method, and a stable structure search program.

### BACKGROUND

In recent years, in the field of drug discovery, drug discovery based on a middle molecule (having a molecular weight of 500 to 3000) that involves less side effects has been expected, and development of a search method for searching for a stable structure of a middle molecule is in progress.

As an example, there is a search method in which a search is performed on a coarse-grained model by using an interaction potential. According to this search method, an initial structure close to a stable structure of a middle molecule may be searched for at a high speed.

Japanese Laid-open Patent Publication No. 2020-173643 and International Publication Pamphlet No. WO 2019/235567 are disclosed as related art.

### SUMMARY

### [TECHNICAL PROBLEM]

However, to apply the search method described above, an interaction potential of a coarse-grained particle (molecule) is to be calculated in advance for a coarse-grained model of a search-target middle molecule. Thus, for example, in a case where a coarse-grained particle (molecule) for which an interaction potential has not been calculated is included in the coarse-grained model as in the case of an unnatural amino acid or the like, the interaction potential of the coarse-grained particle (molecule) is to be calculated first over an enormous period of time, which is inefficient.

In one aspect, an object is to efficiently search for a stable structure of a middle molecule.

### [SOLUTION TO PROBLEM]

According to an aspect of the embodiments, a stable structure search system includes a calculation unit that acquires a plurality of degrees of structure similarity between each of a plurality of kinds of molecules and a first molecule included in a target molecule based on each interaction potential of the plurality of kinds of molecules, acquires a plurality of degrees of charge similarity between each of the plurality of kinds of molecules and the first molecule, and acquires a plurality of total degrees of similarity between each of the plurality of kinds of molecules and the first molecule based on sums of each of the plurality of degrees of structure similarity and each of the plurality of degrees of charge similarity; and an identification unit that determines a second molecule whose total degree of similarly is largest among the plurality of kinds of molecules, acquires energy of a molecular structure of the target molecule based on an interaction potential of the second molecule, and determines whether the calculated energy satisfies a certain condition.

### [ADVANTAGEOUS EFFECT OF INVENTION]

A stable structure of a middle molecule may be efficiently searched for.

### BRIEF DESCRIPTION OF DRAWINGS

FIG. 1 is a diagram illustrating an example of a system configuration of a stable structure search system;
FIG. 2 is a diagram illustrating an example of a hardware configuration of a terminal apparatus;
FIG. 3 is a diagram illustrating an example of single amino acid residue information;
FIG. 4 is a diagram illustrating an example of a functional configuration of a degree-of-similarity calculation information input unit;
FIG. 5 is a diagram illustrating a specific example of processing of a graphing unit;
FIG. 6 is a diagram illustrating a specific example of processing of a conflict graph generation unit;
FIG. 7 is a diagram illustrating a specific example of processing of an interaction potential acquisition unit;
FIG. 8 is a diagram illustrating a specific example of processing of a stable structure search unit;
FIG. 9 is a diagram illustrating a specific example of processing of a degree-of-structure-similarity calculation unit;
FIG. 10 is a diagram illustrating a specific example of processing of a degree-of-charge-similarity calculation unit;
FIG. 11 is a diagram illustrating a specific example of processing of a total-degree-of-similarity calculation unit;
FIG. 12 is a diagram illustrating an effect of calculating a total degree of similarity; and
FIG. 13 is a flowchart illustrating a flow of a stable structure search process.

### DESCRIPTION OF EMBODIMENTS

Each embodiment will be described below with reference to the accompanying drawings. In the present specification and drawings, components having substantially the same functional configurations are denoted by the same reference sign, whereby redundant description will be omitted.

### [First Embodiment]

### <System Configuration of Stable Structure Search System>

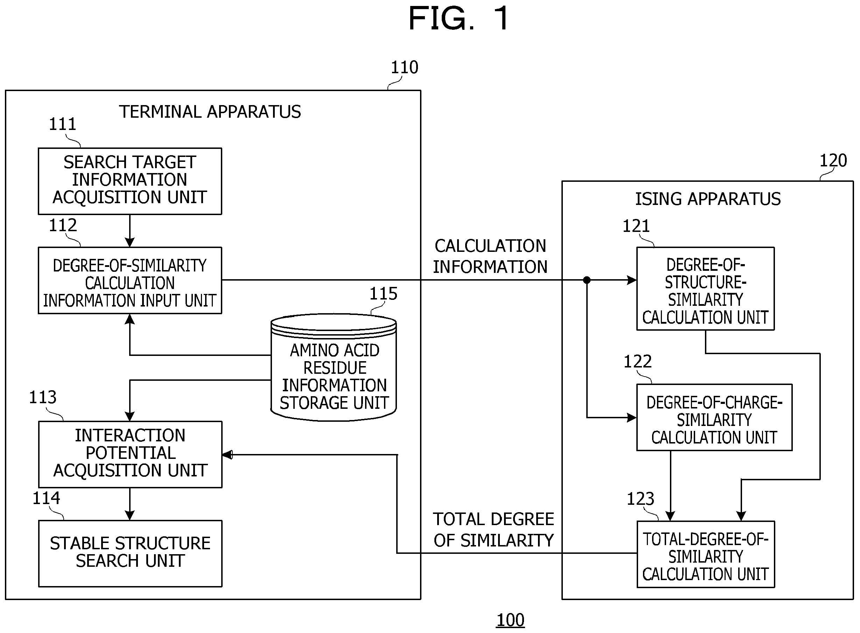

A system configuration of a stable structure search system according to a first embodiment will be described first. FIG. 1 is a diagram illustrating an example of the system configuration of the stable structure search system. A stable structure search system 100 is a system for searching for a stable structure of a middle molecule. In the first embodiment, the stable structure search system 100 implements a high-speed search by performing coarse-graining of a molecular structure of a middle molecule and searching for a stable structure of the coarse-grained model in a lattice space.

An illustrated in FIG. 1, the stable structure search system 100 includes a terminal apparatus 110 and an Ising apparatus 120 such as a quantum annealing apparatus.

A search program is installed on the terminal apparatus 110. As a result of this program being executed, the terminal apparatus 110 functions as a search target information acquisition unit 111, a degree-of-similarity calculation information input unit 112, an interaction potential acquisition unit 113, and a stable structure search unit 114.

The search target information acquisition unit 111 acquires a coarse-grained model of a search-target middle molecule whose stable structure is to be searched for.

In the first embodiment, the coarse-grained model of the search-target middle molecule acquired by the search target information acquisition unit 111 is, for example, a coarse-grained model including a coarse-grained particle (molecule) for which an interaction potential has not been calculated, such as a coarse-grained model of an unnatural amino acid.

The search target information acquisition unit 111 notifies the degree-of-similarity calculation information input unit 112 of the acquired coarse-grained model.

Upon being notified of the coarse-grained model by the search target information acquisition unit 111, the degree-of-similarity calculation information input unit 112 refers to an amino acid residue information storage unit 115. The amino acid residue information storage unit 115 stores information on a plurality of kinds of single amino acid residues, each of which is a single amino acid residue (for example, one natural amino acid residue) that is a coarse-grained particle (molecule) with a known interaction potential.

The degree-of-similarity calculation information input unit 112 sequentially reads the information on the plurality of kinds of coarse-grained particles (molecules) with known interaction potentials stored in the amino acid residue information storage unit 115. The degree-of-similarity calculation information input unit 112 sequentially generates calculation information for use in calculation of a degree of similarity between the coarse-grained particle (molecule) for which the interaction potential has not been calculated and which is included in the notified coarse-grained model and a coarse-grained particle (molecule) with a known interaction potential.

The degree-of-similarity calculation information input unit 112 sequentially inputs the generated calculation information to the Ising apparatus 120, and causes the Ising apparatus 120 to calculate a total degree of similarity (details will be described later) between the coarse-grained particle (molecule) for which the interaction potential has not been calculated and which is included in the coarse-grained model of the search-target middle molecule and each of the plurality of kinds of coarse-grained particles (molecules) with known interaction potentials.

The interaction potential acquisition unit 113 sequentially acquires the individual total degrees of similarity calculated by the Ising apparatus 120 from the Ising apparatus 120. The interaction potential acquisition unit 113 determines a largest total degree of similarity from among the acquired total degrees of similarity, and extracts the coarse-grained particle (molecule) that has a known interaction potential and corresponds to the determined total degree of similarity.

The interaction potential acquisition unit 113 refers to the amino acid residue information storage unit 115, acquires an interaction potential stored in association with the extracted coarse-grained particle (molecule) with the known interaction potential, and notifies the stable structure search unit 114 of the interaction potential.

The stable structure search unit 114 is an example of an identification unit, and calculates, by using the notified interaction potential, energies in various arrangements of the coarse-grained particle (molecule) included in the coarse-grained model of the search-target middle molecule. In this manner, the stable structure search unit 114 identifies, as a stable structure of the coarse-grained model, a molecular structure of the coarse-grained model indicated by the arrangement of the coarse-grained particle (molecule) for which the calculation result of the energy satisfies a predetermined condition.

As described above, in the stable structure search system 100, when the stable structure of the coarse-grained model of the search-target middle molecule is searched for, the known interaction potential of the similar coarse-grained particle (molecule) is used as an alternative to the unknown interaction potential of the coarse-grained particle (molecule) used in the search for the stable structure.

Thus, according to the stable structure search system 100, even if a coarse-grained particle (molecule) for which an interaction potential has not been calculated is included in the coarse-grained model of the search-target middle molecule, calculation of the interaction potential over an enormous period of time may be omitted. As a result, according to the first embodiment, the stable structure of the middle molecule may be efficiently searched for.

On the other hand, a degree-of-similarity calculation program is installed on the Ising apparatus 120. As a result of this program being executed, the Ising apparatus 120 functions as a degree-of-structure-similarity calculation unit 121, a degree-of-charge-similarity calculation unit 122, and a total-degree-of-similarity calculation unit 123.

The degree-of-structure-similarity calculation unit 121 acquires the calculation information from the degree-of-similarity calculation information input unit 112. The calculation information includes degree-of-structure-similarity calculation information for use in calculation of a degree of structure similarity. Based on this degree-of-structure-similarity calculation information, the degree-of-structure-similarity calculation unit 121 calculates a degree of structure similarity between a coarse-grained particle (molecule) for which the interaction potential has not been calculated and which is included in the coarse-grained model of the search-target middle molecule and each of a plurality of kinds of coarse-grained particles (molecules) with known interaction potentials.

The degree-of-structure-similarity calculation unit 121 notifies the total-degree-of-similarity calculation unit 123 of the calculated degree of structure similarity.

The degree-of-charge-similarity calculation unit 122 acquires the calculation information from the degree-of-similarity calculation information input unit 112. The calculation information includes degree-of-charge-similarity calculation information for use in calculation of a degree of charge similarity. Based on this degree-of-charge-similarity calculation information, the degree-of-charge-similarity calculation unit 122 calculates a degree of charge similarity between the coarse-grained particle (molecule) for which the interaction potential has not been calculated and which is included in the coarse-grained model of the search-target middle molecule and each of the plurality of kinds of coarse-grained particles (molecules) with known interaction potentials.

The degree-of-charge-similarity calculation unit 122 notifies the total-degree-of-similarity calculation unit 123 of the calculated degree of charge similarity.

The total-degree-of-similarity calculation unit 123 is an example of a calculation unit, and calculates a total degree of similarity by performing weighted addition of the degree of structure similarity notified by the degree-of-structure-similarity calculation unit 121 and the degree of charge similarity notified by the degree-of-charge-similarity calculation unit 122. The total-degree-of-similarity calculation unit 123 transmits the calculated total degree of similarity to the interaction potential acquisition unit 113 of the terminal apparatus 110.

### <Hardware Configuration of Terminal Apparatus>

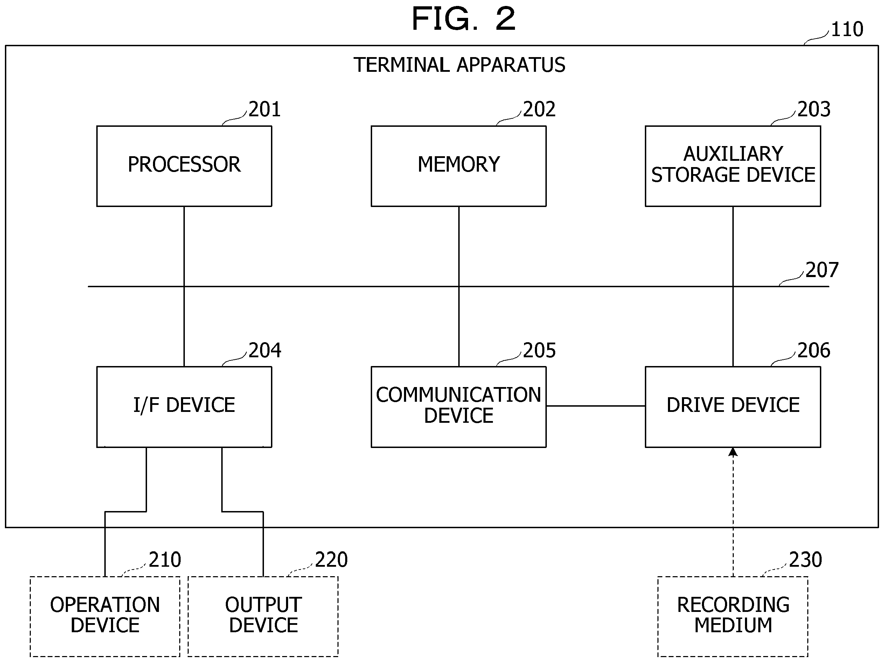

A hardware configuration of the terminal apparatus 110 will be described next. FIG. 2 is a diagram illustrating an example of the hardware configuration of the terminal apparatus 110.

As illustrated in FIG. 2, the terminal apparatus 110 includes a processor 201, a memory 202, an auxiliary storage device 203, an interface (I/F) device 204, a communication device 205, and a drive device 206. The individual pieces of hardware of the terminal apparatus 110 are coupled to each other by a bus 207.

The processor 201 includes various computing devices such as a central processing unit (CPU) and a graphics processing unit (GPU). The processor 201 reads various programs (for example, the search program and the like) to the memory 202 and executes the programs.

The memory 202 includes main storage devices such as a read-only memory (ROM) and a random-access memory (RAM). The processor 201 and the memory 202 form a so-called computer. As a result of the processor 201 executing the various programs read to the memory 202, the computer implements various functions.

The auxiliary storage device 203 stores the various programs and various kinds of information used when the various programs are executed by the processor 201.

The I/F device 204 is a coupling device that couples an operation device 210 and an output device 220, which are an example of external devices, and the terminal apparatus 110 to each other.

The communication device 205 is a communication device for communicating with the Ising apparatus 120, which is an example of another apparatus.

The drive device 206 is a device in which a recording medium 230 is to be placed. Examples of the recording medium 230 herein include a medium on which information is recorded optically, electrically, or magnetically, such as a compact disc read-only memory (CD-ROM), a flexible disk, or a magneto-optical disk. Examples of the recording medium 230 may also include a semiconductor memory or the like on which information is recorded electrically, such as a ROM or a flash memory.

The various programs to be installed onto the auxiliary storage device 203 are installed, for example, in such a way that the distributed recording medium 230 is placed in the drive device 206 and the drive device 206 reads the various programs recorded on the recording medium 230. Alternatively, the various programs to be installed onto the auxiliary storage device 203 may be installed in such a way that the various programs are downloaded from a network via the communication device 205.

Although only the hardware configuration of the terminal apparatus 110 is described and the hardware configuration of the Ising apparatus 120 is not described herein, the hardware configuration of the Ising apparatus 120 may be substantially the same as that of the terminal apparatus 110, for example. Alternatively, the hardware configuration of the Ising apparatus 120 may be substantially the same as the hardware configuration of a so-called quantum computer.

### <Specific Example of Single Amino Acid Residue Information>

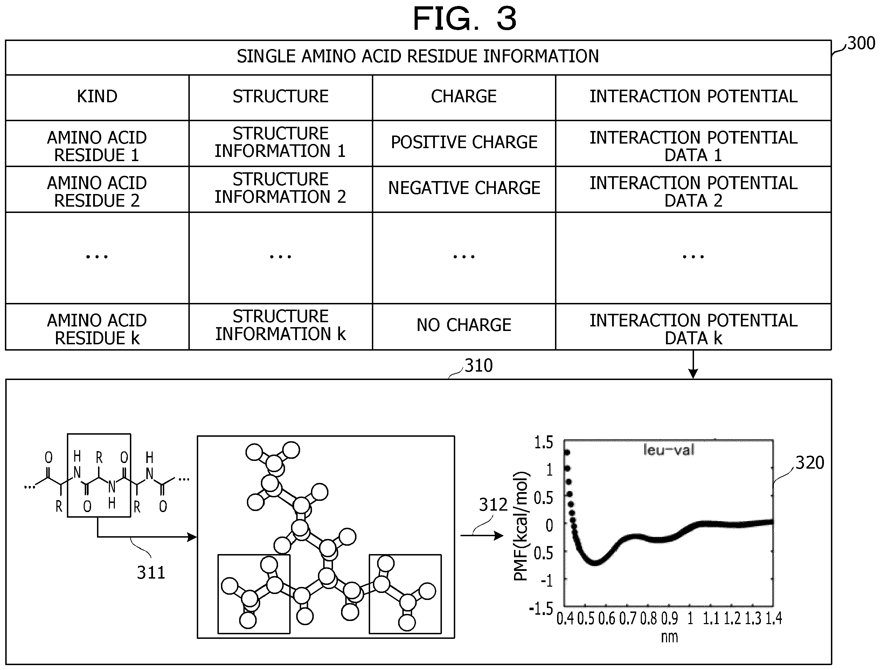

A specific example of information on a plurality of kinds of single amino acid residues stored in the amino acid residue information storage unit 115 will be described next. FIG. 3 is a diagram illustrating an example of single amino acid residue information. As illustrated in FIG. 3, single amino acid residue information 300 includes "kind", "structure", "charge", and "interaction potential" as information items.

At "kind", a plurality of kinds of single natural amino acid residues, which are coarse-grained particles (molecules) with known interaction potentials, are stored. The example of FIG. 3 indicates a state in which k kinds of single natural amino acid residues, which are coarse-grained particles (molecules) with known interaction potentials, are stored.

At "structure", structure information of the corresponding kind of single natural amino acid residue is stored. At "charge", charge information of the corresponding kind of single natural amino acid residue is stored.

At "interaction potential", interaction potential data calculated in advance for the corresponding kind of single natural amino acid residue is stored.

In FIG. 3, a reference sign 310 indicates an overview of a method for calculating interaction potential data calculated in advance for a predetermined kind of single natural amino acid residue. As indicated by the reference sign 310, to calculate the interaction potential data, a single amino acid residue is taken out from the predetermined kind of natural amino acid, a terminal portion is treated by imitating a protein, and a force field is created so as not to cause interaction (see an arrow 311).

Subsequently, as indicated by the reference sign 310, for the taken-out single amino acid residue, molecular dynamics calculation at each intermolecular distance is performed by using umbrella sampling. Subsequently, as indicated by the reference sign 310, the sampling results at the respective distances are integrated by using the weighted histogram analysis method, so that interaction potential data is generated (see an arrow 312).

In FIG. 3, a graph 320 visually represents the generated interaction potential data. The horizontal axis represents each intermolecular distance, and the vertical axis represents energy.

### <Specific Example of Processing of Each Unit of Terminal Apparatus>

A specific example of processing of each of the units (the degree-of-similarity calculation information input unit 112, the interaction potential acquisition unit 113, and the stable structure search unit 114 in this case) of the terminal apparatus 110 will be described next.

### (1) Specific Example of Processing of Degree-of-Similarity Calculation Information Input Unit

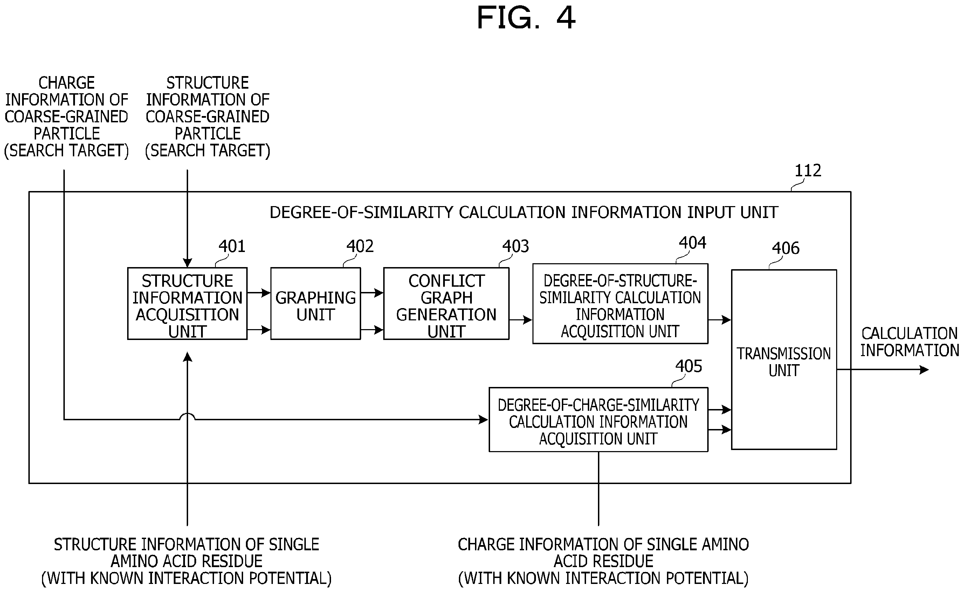

A specific example of processing of the degree-of-similarity calculation information input unit 112 will be described first. FIG. 4 is a diagram illustrating an example of a functional configuration of the degree-of-similarity calculation information input unit 112. As illustrated in FIG. 4, the degree-of-similarity calculation information input unit 112 includes a structure information acquisition unit 401, a graphing unit 402, a conflict graph generation unit 403, a degree-of-structure-similarity calculation information acquisition unit 404, a degree-of-charge-similarity calculation information acquisition unit 405, and a transmission unit 406.

The structure information acquisition unit 401 acquires structure information of a coarse-grained particle (molecule) included in the coarse-grained model of the search-target middle molecule acquired by the search target information acquisition unit 111. The structure information acquisition unit 401 also acquires structure information of each of the plurality of kinds of single amino acid residues, which are coarse-grained particles (molecules) with known interaction potentials, read from the amino acid residue information storage unit 115.

The graphing unit 402 graphs the structure information of the coarse-grained particle (molecule) included in the coarse-grained model of the search-target middle molecule notified by the search target information acquisition unit 111. The graphing unit 402 graphs the structure information of each of the plurality of kinds of single amino acid residues, which are coarse-grained particles (molecules) with known interaction potentials. Graphing performed by the graphing unit 402 means, for example, presenting the structure of the coarse-grained particle (molecule) by using information on kinds of atoms (elements) included in the coarse-grained particle (molecule) and information on a bonding state of the individual atoms.

The conflict graph generation unit 403 creates vertices (nodes) of a conflict graph by combining vertices (atoms) in the graphed coarse-grained particles (molecules) with each other. The conflict graph generation unit 403 compares the generated nodes with each other, and creates an edge between the nodes depending on whether or not the nodes are constituted by atoms in different states.

The degree-of-structure-similarity calculation information acquisition unit 404 acquires, as the degree-of-structure-similarity calculation information, each of the conflict graphs generated by the conflict graph generation unit 403. In a conflict graph, determining a set (maximum independent set) including a largest number of nodes among sets each constituted by nodes without any edge therebetween is synonymous to determining a largest partial structure among partial structures common to two coarse-grained particles (molecules). For example, the conflict graph acquired by the degree-of-structure-similarity calculation information acquisition unit 404 is used by the degree-of-structure-similarity calculation unit 121 to determine the largest partial structure (for example, the degree of structure similarity) among the partial structures common to the two coarse-grained particles (molecules).

The degree-of-charge-similarity calculation information acquisition unit 405 acquires charge information of the coarse-grained particle (molecule) included in the coarse-grained model of the search-target middle molecule acquired by the search target information acquisition unit 111. The degree-of-charge-similarity calculation information acquisition unit 405 also acquires charge information of each of the plurality of kinds of single amino acid residues, which are coarse-grained particles (molecules) with known interaction potentials, read from the amino acid residue information storage unit 115.

The degree-of-charge-similarity calculation information acquisition unit 405 notifies the transmission unit 406 of the acquired charge information of each of the plurality of kinds of coarse-grained particles (molecules) as the degree-of-charge-similarity calculation information.

The transmission unit 406 transmits, as the calculation information, the degree-of-structure-similarity calculation information acquired by the degree-of-structure-similarity calculation information acquisition unit 404 and the degree-of-charge-similarity calculation information acquired by the degree-of-charge-similarity calculation information acquisition unit 405 to the Ising apparatus 120.

### a) Details of Processing of Graphing Unit

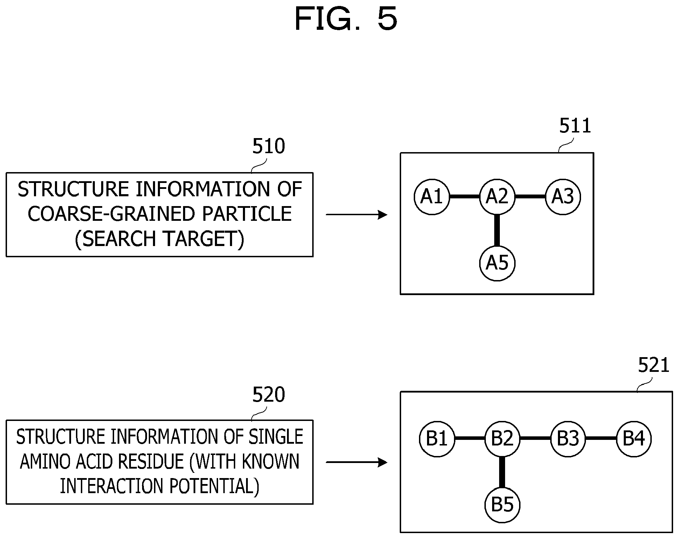

Details of the processing of the graphing unit 402 will be described next. FIG. 5 is a diagram illustrating a specific example of the processing of the graphing unit 402. Upon acquiring structure information 510 of the coarse-grained particle (molecule) included in the coarse-grained model of the search-target middle molecule, the graphing unit 402 generates a graph 511. An example of the structure information 510 in FIG. 5 indicates that the coarse-grained particle (molecule) is formed by four atoms, which are bonded in a bonding state as illustrated in the graph 511.

Likewise, upon acquiring structure information 520 of a single amino acid residue that is a coarse-grained particle (molecule) with a known interaction potential, the graphing unit 402 generates a graph 521. An example of the structure information 520 in FIG. 5 indicates that the coarse-grained particle (molecule) is formed by five atoms, which are bonded in a bonding state as illustrated in the graph 521.

### b) Details of Processing of Conflict Graph Generation Unit

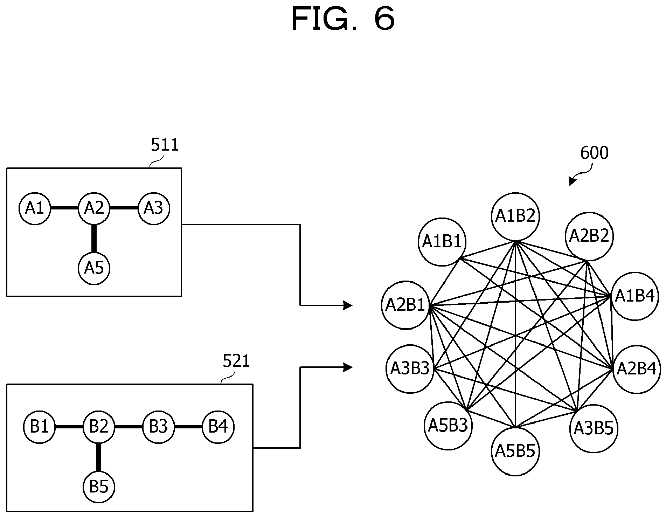

Details of the processing of the conflict graph generation unit 403 will be described next. FIG. 6 is a diagram illustrating a specific example of the processing of the conflict graph generation unit 403. For example, upon acquiring the graph 511 and the graph 521 from the graphing unit 402, the conflict graph generation unit 403 combines the atoms in the individual graphs and creates vertices (nodes) of a conflict graph. The example in FIG. 6 indicates that ten nodes are created.

Subsequently, the conflict graph generation unit 403 creates a conflict graph 600 by determining whether or not to create an edge based on a rule of creating an edge between nodes if the nodes are constituted by atoms in different states and a rule of not creating an edge between nodes if the nodes are constituted by atoms in the same state.

The conflict graph 600 created by the conflict graph generation unit 403 is acquired by the degree-of-structure-similarity calculation information acquisition unit 404 and is transmitted to the Ising apparatus 120 by the transmission unit 406, as the degree-of-structure-similarity calculation information.

As described above, in the conflict graph 600, determining a maximum independent set among sets each constituted by nodes without any edge therebetween is synonymous to determining a largest partial structure among partial structures common to two coarse-grained particles (molecules). Thus, the degree-of-structure-similarity calculation unit 121 of the Ising apparatus 120 to which the conflict graph 600 is transmitted calculates the degree of structure similarity by determining the largest partial structure among the partial structures common to the two coarse-grained particles (molecules).

### (2) Specific Example of Processing of Interaction Potential Acquisition Unit

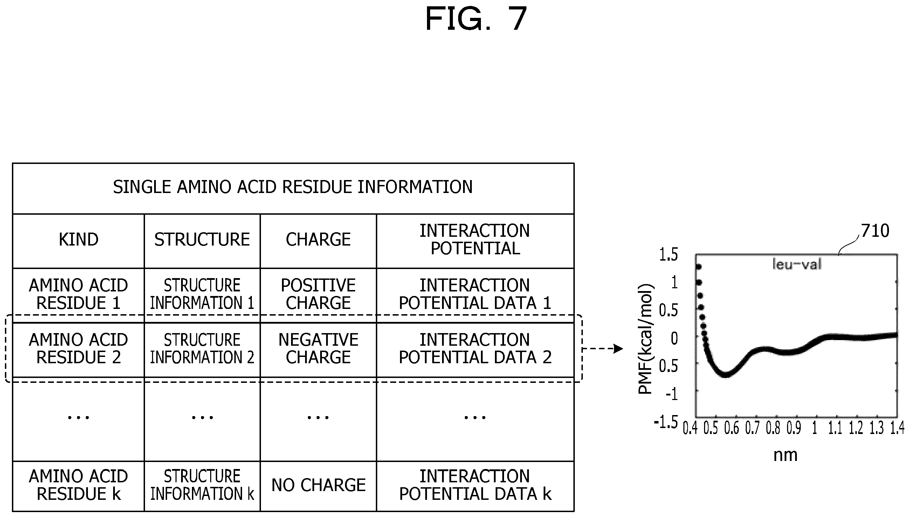

A specific example of processing of the interaction potential acquisition unit 113 will be described next. FIG. 7 is a diagram illustrating a specific example of the processing of the interaction potential acquisition unit 113.

The example in FIG. 7 indicates a state in which a largest total degree of similarity is determined from the total degrees of similarity acquired from the Ising apparatus 120 and "kind" = "amino acid residue 2" is extracted as the single amino acid residue that is a coarse-grained particle (molecule) corresponding to the determined largest total degree of similarity.

As the "interaction potential" of the extracted "amino acid residue 2", the interaction potential acquisition unit 113 reads "interaction potential data 2" from the amino acid residue information storage unit 115 and notifies the stable structure search unit 114 of the "interaction potential data 2". A reference sign 710 represents a specific example of the "interaction potential data 2" that is the "interaction potential" of the "amino acid residue 2".

### (3) Specific Example of Processing of Stable Structure Search Unit

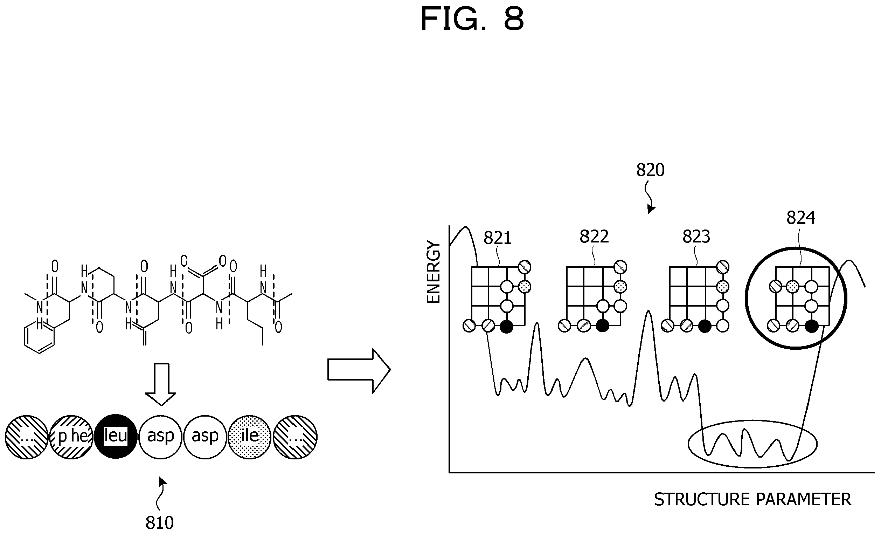

A specific example of processing of the stable structure search unit 114 will be described next. FIG. 8 is a diagram illustrating a specific example of the processing of the stable structure search unit 114. In FIG. 8, a reference sign 810 represents the coarse-grained model of the search-target middle molecule. For simplification of description, the example in FIG. 8 indicates a case where the coarse-grained model of the search-target middle molecule is constituted by seven coarse-grained particles (molecules).

In FIG. 8, a reference sign 820 represents a result obtained by the stable structure search unit 114 calculating energies in various arrangements of the coarse-grained particles (molecules) in a lattice space by using the "interaction potential data 2" acquired by the interaction potential acquisition unit 113.

According to the reference sign 820, energy in an arrangement 824 is lower than energies in arrangements 821, 822, and 823. Thus, the stable structure search unit 114 outputs the arrangement 824 as the search result (the stable structure of the coarse-grained model) for the coarse-grained model (the reference sign 810) of the search-target middle molecule.

### <Specific Example of Processing of Each Unit of Ising Apparatus>

A specific example of processing of each of the units (the degree-of-structure-similarity calculation unit 121, the degree-of-charge-similarity calculation unit 122, and the total-degree-of-similarity calculation unit 123) of the Ising apparatus 120 will be described next.

### (1) Specific Example of Processing of Degree-of-Structure-Similarity Calculation Unit

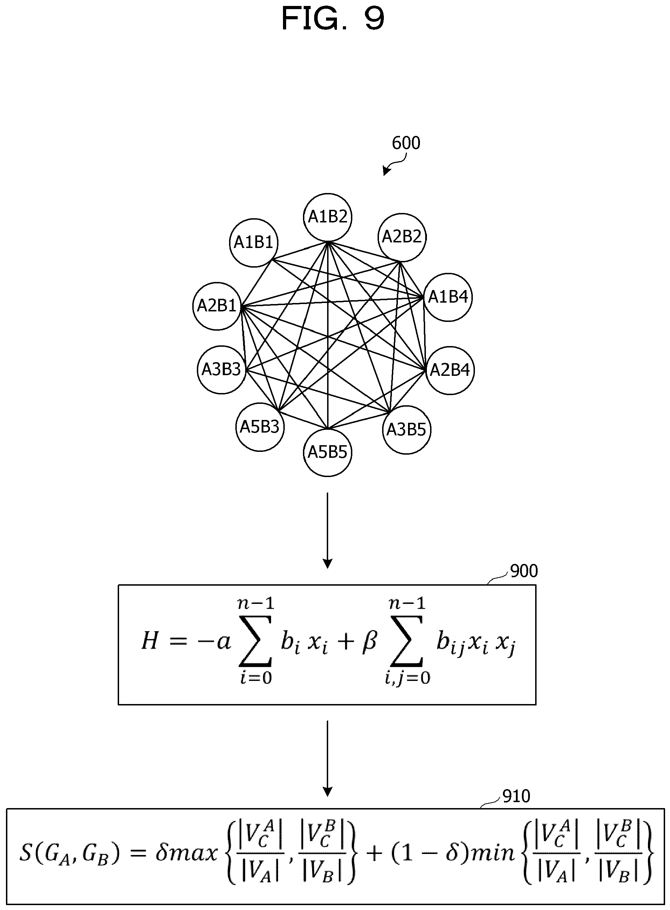

A specific example of processing of the degree-of-structure-similarity calculation unit 121 will be described first. FIG. 9 is a diagram illustrating a specific example of the processing of the degree-of-structure-similarity calculation unit 121.

As illustrated in FIG. 9, the maximum independent set of the conflict graph 600 may be determined by using, for example, the Hamiltonian whose minimization means searching for the maximum independent set.

In FIG. 9, a reference sign 900 represents an equation representing the Hamiltonian (H), where n denotes the number of nodes in the conflict graph 600, bᵢ denotes a numerical value representing a bias for an i-th node, bᵢⱼ denotes a positive number that is not 0 if there is an edge between the i-th node and a j-th node and denotes 0 if there is no edge, xᵢ denotes a binary variable indicating that the i-th node is 0 or 1, xⱼ denotes a binary variable indicating that the j-th node is 0 or 1, and α and β denote positive values.

By determining the maximum independent set by using the equation indicating the Hamiltonian (H) (the reference sign 900), the degree-of-structure-similarity calculation unit 121 determines the largest partial structure common to the coarse-grained particle (molecule) for which the interaction potential has not been calculated and which is included in the coarse-grained model of the search-target middle molecule and each of the plurality of kinds of coarse-grained particles (molecules) with known interaction potentials.

Subsequently, the degree-of-structure-similarity calculation unit 121 calculates the degree of structure similarity, based on the determined maximum independent set. A reference sign 910 in FIG. 9 represents an equation for calculating a degree of structure similarity S(G_{A}, G_{B}), where V^{A} denotes the total number of nodes in the graphed coarse-grained model of the search-target middle molecule, V_{C}^{A} denotes the number of nodes included in the maximum independent set of the conflict graph 600 among the nodes in the graphed coarse-grained model of the search-target middle molecule, V^{B} denotes the total number of nodes in the graphed coarse-grained model with a known interaction potential, V_{C}^{B} denotes the number of nodes included in the maximum independent set of the conflict graph 600 among the nodes in the graphed coarse-grained model with the known interaction potential, and δ denotes a value from 0 to 1.

### (2) Specific Example of Processing of Degree-of-Charge-Similarity Calculation Unit

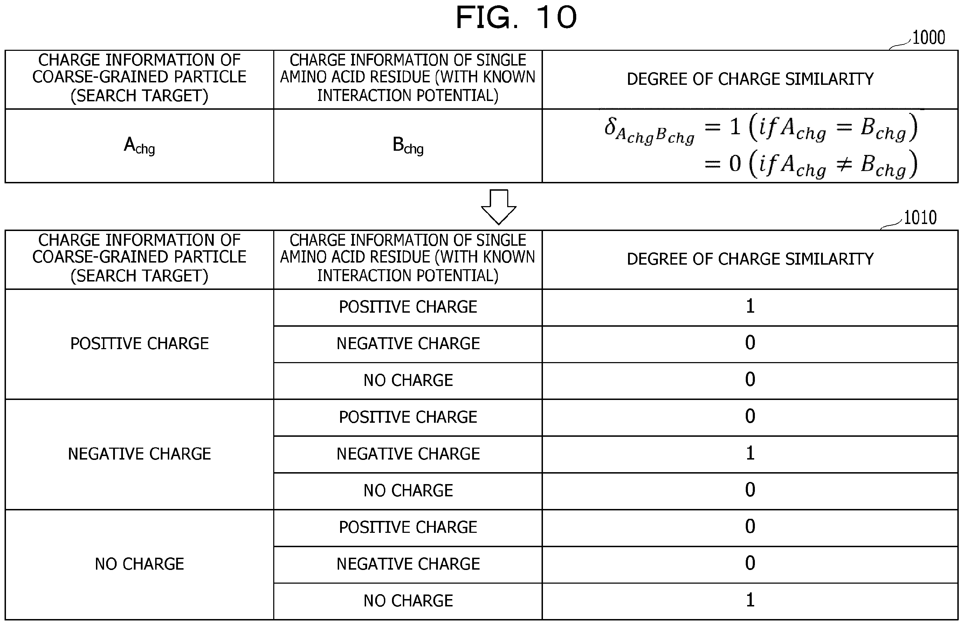

A specific example of processing of the degree-of-charge-similarity calculation unit 122 will be described next. FIG. 10 is a diagram illustrating a specific example of the processing of the degree-of-charge-similarity calculation unit 122.

In FIG. 10, a table 1000 presents a method for calculating a degree of charge similarity performed by the degree-of-charge-similarity calculation unit 122. As indicated by the table 1000, the charge type of the coarse-grained particle (molecule) included in the coarse-grained model of the search-target middle molecule is denoted by A_{chg} and the charge type of the single amino acid residue that is the coarse-grained particle (molecule) with a known interaction potential is denoted by B_{chg}. In this case, the degree-of-charge-similarity calculation unit 122 calculates the degree of charge similarity δ_{AchgBchg} to be equal to 1 if the charge type of the coarse-grained particle (molecule) for which the interaction potential has not been calculated and which is included in the coarse-grained model of the search-target middle molecule is the same as the charge type of the single amino acid residue that is the coarse-grained particle (molecule) with a known interaction potential, and to be equal to 0 if the charge type of the coarse-grained particle (molecule) for which the interaction potential has not been calculated and which is included in the coarse-grained model of the search-target middle molecule is different from the charge type of the single amino acid residue that is the coarse-grained particle (molecule) with the known interaction potential.

In FIG. 10, a table 1010 presents a specific example of the degree of charge similarity calculated by the degree-of-charge-similarity calculation unit 122.

As indicated by the table 1010, in a case where the charge type A_{chg} of the coarse-grained particle (molecule) for which the interaction potential has not been calculated and which is included in the coarse-grained model of the search-target middle molecule is positive charge, the degree of charge similarity is calculated to be equal to 1 if the charge type B_{chg} of the coarse-grained model of the single amino acid residue that is the coarse-grained particle (molecule) with a known interaction potential is positive charge, to be equal to 0 if the charge type B_{chg} is negative charge, and to be equal to 0 if the charge type B_{chg} is no charge. In a case where the charge type A_{chg} of the coarse-grained particle (molecule) for which the interaction potential has not been calculated and which is included in the coarse-grained model of the search-target middle molecule is negative charge, the degree of charge similarity is calculated to be equal to 0 if the charge type B_{chg} of the coarse-grained model of the single amino acid residue that is the coarse-grained particle (molecule) with a known interaction potential is positive charge, to be equal to 1 if the charge type B_{chg} is negative charge, and to be equal to 0 if the charge type B_{chg} is no charge. In a case where the charge type A_{chg} of the coarse-grained particle (molecule) for which the interaction potential has not been calculated and which is included in the coarse-grained model of the search-target middle molecule is no charge, the degree of charge similarity is calculated to be equal to 0 if the charge type B_{chg} of the coarse-grained model of the single amino acid residue that is the coarse-grained particle (molecule) with a known interaction potential is positive charge, to be equal to 0 if the charge type B_{chg} is negative charge, and to be equal to 1 if the charge type B_{chg} is no charge.

### (3) Specific Example of Processing of Total-Degree-of-Similarity Calculation Unit

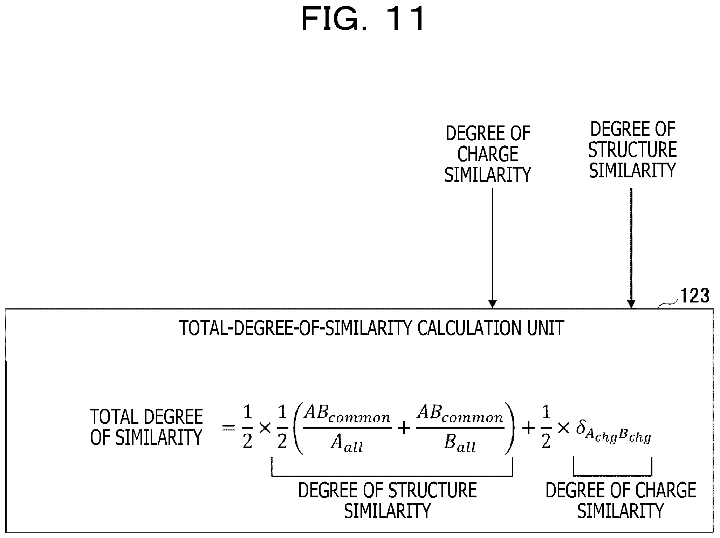

A specific example of processing of the total-degree-of-similarity calculation unit 123 will be described next. FIG. 11 is a diagram illustrating a specific example of the processing of the total-degree-of-similarity calculation unit 123. As illustrated in FIG. 11, the total-degree-of-similarity calculation unit 123 calculates the total degree of similarity by performing weighted addition of the degree of structure similarity and the degree of charge similarity. In FIG. 11, 1/2×(AB_{common/}Aₐₗₗ + AB_{common/}Bₐₗₗ) and δ_{AchgBchg} denote the degree of structure similarity and the degree of charge similarity, respectively. The example in FIG. 11 indicates a case where weighted addition is performed on the degree of structure similarity and the degree of charge similarity by using a weighting coefficient of 1/2.

### <Effect of Calculating Total Degree of Similarity>

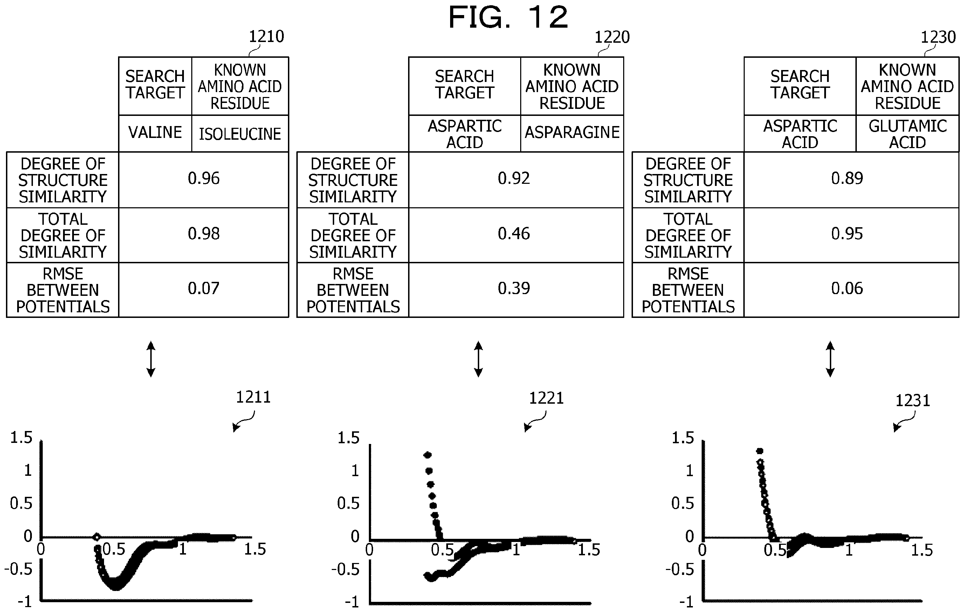

An effect of calculating the total degree of similarity by the Ising apparatus 120 will be examined next. FIG. 12 is a diagram illustrating an effect of calculating the total degree of similarity. In the example in FIG. 12, for two kinds of coarse-grained particles (molecules) with known interaction potentials, a calculation result of the degree of structure similarity, a calculation result of the total degree of similarity, and an error in interaction potential data between the two kinds of coarse-grained particles (molecules) are presented, so that which of the calculation result of the degree of structure similarity and the calculation result of the total degree of similarity more accurately represents a degree of similarity between the interaction potentials is examined.

Among these, a reference sign 1210 presents an examined result obtained in a case where the coarse-grained particle (molecule) included in the coarse-grained model of the search-target middle molecule is assumed to be "valine" and the coarse-grained particle (molecule) with a known interaction potential is assumed to be "isoleucine". According to the reference sign 1210, the degree of structure similarity between "valine" and "isoleucine" is "0.96" and the total degree of similarity therebetween is "0.98". On the other hand, an error (root mean square error: RMSE) between the interaction potential data of "valine" and the interaction potential data of "isoleucine" is "0.07", and their interaction potentials are similar (see a graph 1211).

Thus, in a case of the reference sign 1210, it may be said that both the calculation result of the degree of structure similarity and the calculation result of the total degree of similarity accurately represent the degree of similarity between the interaction potentials.

In contrast, a reference sign 1220 presents an examined result obtained in a case where the coarse-grained particle (molecule) included in the coarse-grained model of the search-target middle molecule is assumed to be "aspartic acid" and the coarse-grained particle (molecule) with a known interaction potential is assumed to be "asparagine". According to the reference sign 1220, the degree of structure similarity between "aspartic acid" and "asparagine" is "0.92" but the total degree of similarity therebetween is "0.46". On the other hand, an error (RMSE) between the interaction potential data of "aspartic acid" and the interaction potential data of "asparagine" is "0.39", and their interaction potentials are not similar (see a graph 1221).

Thus, in a case of the reference sign 1220, it is not said that the calculation result of the degree of structure similarity accurately represents the degree of similarity between the interaction potentials. In contrast, it may be said that the calculation result of the total degree of similarity accurately represents the degree of similarity between the interaction potentials.

A reference sign 1230 presents an examined result obtained in a case where the coarse-grained particle (molecule) included in the coarse-grained model of the search-target middle molecule is assumed to be "aspartic acid" and the coarse-grained particle (molecule) with a known interaction potential is assumed to be "glutamic acid". According to the reference sign 1230, the degree of structure similarity between "aspartic acid" and "glutamic acid" is "0.89" and the total degree of similarity therebetween is "0.95". On the other hand, an error (RMSE) between the interaction potential data of "aspartic acid" and the interaction potential data of "glutamic acid" is "0.06", and their interaction potentials are similar (see a graph 1231).

According to the comparison between the case of the reference sign 1220 and the case of the reference sign 1230, in a case where the coarse-grained particle (molecule) included in the coarse-grained model of the search-target middle molecule is "aspartic acid", if the determination is made only based on the degree of structure similarity, "asparagine" is output as the search result instead of "glutamic acid". This is because there is a relationship of the degree of structure similarity of "asparagine" (= 0.92) > the degree of structure similarity of "glutamic acid" (= 0.89).

However, as is apparent from the graph 1221, the interaction potential of "aspartic acid" and the interaction potential of "asparagine" are not similar. For example, if determination is made only based on the degree of structure similarity, an incorrect search result is output.

On the other hand, if determination is made based on the total degree of similarity, "glutamic acid" is output as the search result. This is because there is a relationship of the total degree of similarity of "asparagine" (= 0.46) < the total degree of similarity of "glutamic acid" (= 0.95).

As is apparent from the graph 1231, the interaction potential of "aspartic acid" and the interaction potential of "glutamic acid" are similar. For example, if determination is made by using the total degree of similarity, a correct search result is output.

### <Flow of Stable Structure Search Process>

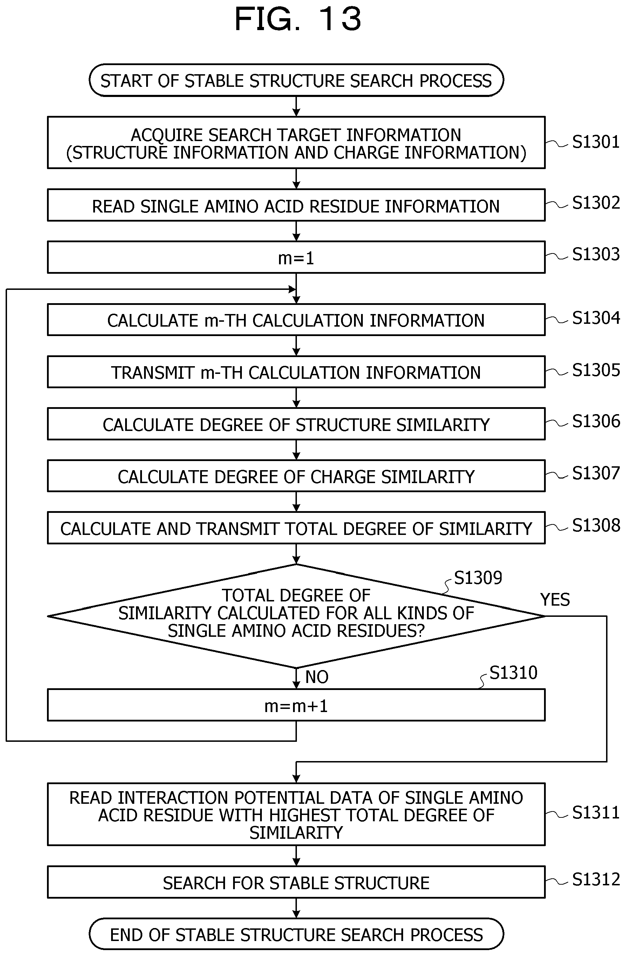

A flow of a stable structure search process performed by the stable structure search system 100 will be described next. FIG. 13 is a flowchart illustrating the flow of the stable structure search process.

In step S1301, the terminal apparatus 110 acquires, as search target information, the structure information of the coarse-grained particle (molecule) included in the coarse-grained model of the search-target middle molecule and the charge information of the coarse-grained particle (molecule) included in the coarse-grained model of the search-target middle molecule.

In step S1302, the terminal apparatus 110 reads the single amino acid residue information 300 from the amino acid residue information storage unit 115.

In step S1303, the terminal apparatus 110 inputs 1 to a counter m that counts the number of kinds of single amino acid residues included in the single amino acid residue information 300.

In step S1304, the terminal apparatus 110 calculates m-th calculation information (the degree-of-structure-similarity calculation information and the degree-of-charge-similarity calculation information), based on the search target information and the structure information and charge information of an m-th single amino acid residue.

In step S1305, the terminal apparatus 110 transmits the m-th calculation information to the Ising apparatus 120.

In step S1306, based on the m-th calculation information received from the terminal apparatus 110, the Ising apparatus 120 calculates the degree of structure similarity between the coarse-grained particle (molecule) for which the interaction potential has not been calculated and which is included in the coarse-grained model of the search-target middle molecule and the m-th single amino acid residue with a known interaction potential.

In step S1307, based on the m-th calculation information received from the terminal apparatus 110, the Ising apparatus 120 calculates the degree of charge similarity between the coarse-grained particle (molecule) for which the interaction potential has not been calculated and which is included in the coarse-grained model of the search-target middle molecule and the m-th single amino acid residue with the known interaction potential.

In step S1308, the Ising apparatus 120 calculates the total degree of similarity, based on the degree of structure similarity and the degree of charge similarity, and transmits the total degree of similarity to the terminal apparatus 110.

In step S1309, the terminal apparatus 110 determines whether or not the total degree of similarity has been calculated for all kinds of single amino acid residues with known interaction potentials.

In step S1309, if it is determined that there is a single amino acid residue with a known interaction potential for which the total degree of similarity is not calculated (in a case of NO in step S1309), the process proceeds to step S1310.

In step S1310, the terminal apparatus 110 increments the counter m that counts the number of kinds of single amino acid residues with known interaction potentials. The process then returns to step S1304.

On the other hand, if it is determined in step S1309 that the total degree of similarity has been calculated for all the kinds of single amino acid residues with known interaction potentials (in a case of YES in step S1309), the process proceeds to step S1311.

In step S1311, the terminal apparatus 110 extracts the single amino acid residue corresponding to the largest total degree of similarity from the plurality of total degrees of similarity transmitted from the Ising apparatus 120. The terminal apparatus 110 refers to the amino acid residue information storage unit 115 and reads the interaction potential data associated with the extracted single amino acid residue.

In step S1312, the terminal apparatus 110 searches for the stable structure of the coarse-grained model of the search-target middle molecule by using the read interaction potential data. The terminal apparatus 110 outputs the molecular structure (arrangement of the coarse-grained particles (molecules)) of the coarse-grained model having the stable structure obtained by the search.

As is apparent from the above description, the stable structure search system 100 according to the first embodiment calculates the degree of structure similarity between each of a plurality of kinds of coarse-grained particles (molecules) with known interaction potentials and a coarse-grained particle (molecule (corresponding to a first molecule)) included in a coarse-grained model of a search-target middle molecule.

The stable structure search system 100 calculates the degree of charge similarity between each of the plurality of kinds of coarse-grained particles (molecules) with known interaction potentials and the coarse-grained particle (molecule (corresponding to the first molecule)) included in the coarse-grained model of the search-target middle molecule.

The stable structure search system 100 performs weighted addition of the degree of structure similarity and the degree of charge similarity. Based on a result of the weighted addition, the stable structure search system 100 extracts one coarse-grained particle (molecule (corresponding to a second molecule)) from the plurality of kinds of coarse-grained particles (molecules) with known interaction potentials.

By using the interaction potential of the extracted coarse-grained particle (molecule (corresponding to the second molecule)), the stable structure search system 100 calculates energies in various arrangements of the coarse-grained particle (molecule (corresponding to the first molecule)) included in the coarse-grained model. The stable structure search system 100 identifies a molecular structure of the coarse-grained model of the search-target middle molecule for which the calculated energy satisfies a predetermined condition.

As described above, the stable structure search system 100 uses, in searching for the stable structure of the coarse-grained model of the search-target middle molecule, the interaction potential of the coarse-grained particle (molecule) having a structure and charge that are similar to those of the coarse-grained particle (molecule) included in the coarse-grained model of the search-target middle molecule. Thus, according to the stable structure search system 100, even if a coarse-grained particle (molecule) for which an interaction potential has not been calculated is included in the coarse-grained model of the search-target middle molecule, calculation of the interaction potential may be omitted. Therefore, an enormous period of time taken for calculating the interaction potential may be reduced.

As a result, according to the first embodiment, the stable structure of the middle molecule may be efficiently searched for.

### [Second Embodiment]

In the first embodiment described above, the description has been given on the assumption that the stable structure search system 100 includes the terminal apparatus 110 and the Ising apparatus 120. However, the stable structure search system 100 may be formed by an apparatus in addition to the terminal apparatus 110 and the Ising apparatus 120 (for example, by three or more apparatuses). Alternatively, the stable structure search system 100 may be formed by an integrated apparatus (for example, one apparatus). In this case, the stable structure search process (FIG. 13) is implemented as a result of this one apparatus executing a stable structure search program (the search program + the degree-of-similarity calculation program).

In the first embodiment described above, the description has been given on the assumption that the terminal apparatus 110 includes the search target information acquisition unit 111 to the stable structure search unit 114 and the Ising apparatus 120 includes the degree-of-structure-similarity calculation unit 121 to the total-degree-of-similarity calculation unit 123. However, some of the functions of the terminal apparatus 110 may be implemented in the Ising apparatus 120. Likewise, some of the functions of the Ising apparatus 120 may be implemented in the terminal apparatus 110.

## Claims

1. A stable structure search system comprising:
a calculation unit that
acquires a plurality of degrees of structure similarity between each of a plurality of kinds of molecules and a first molecule included in a target molecule based on each interaction potential of the plurality of kinds of molecules,
acquires a plurality of degrees of charge similarity between each of the plurality of kinds of molecules and the first molecule, and
acquires a plurality of total degrees of similarity between each of the plurality of kinds of molecules and the first molecule based on sums of each of the plurality of degrees of structure similarity and each of the plurality of degrees of charge similarity; and
an identification unit that
determines a second molecule whose total degree of similarly is largest among the plurality of kinds of molecules,
acquires energy of a molecular structure of the target molecule based on an interaction potential of the second molecule, and
determines whether the calculated energy satisfies a certain condition.

2. The stable structure search system according to claim 1, wherein the identification unit further
acquires the energy based on a coarse-grained model obtained through coarse-graining of the target molecule.

3. The stable structure search system according to claim 1,
wherein the second molecule is a single amino acid residue.

4. The stable structure search system according to claim 2, wherein the calculation unit further
acquires the plurality of degrees of structure similarity by determining a largest partial structure among partial structures common to the first molecule and each of the plurality of kinds of molecules.

5. The stable structure search system according to claim 4, wherein the calculation unit further
acquires the degree of structure similarity based on a conflict graph that is generated based on the coarse-grained model.

6. The stable structure search system according to claim 2, wherein the calculation unit further
acquires the plurality of degrees of charge similarity based on a charge type of the first molecule and a charge type of each of the plurality of kinds of molecules.

7. A stable structure search method for a computer to execute a process comprising:
acquiring a plurality of degrees of structure similarity between each of a plurality of kinds of molecules and a first molecule included in a target molecule based on each interaction potential of the plurality of kinds of molecules;
acquiring a plurality of degrees of charge similarity between each of the plurality of kinds of molecules and the first molecule;
acquiring a plurality of total degrees of similarity between each of the plurality of kinds of molecules and the first molecule based on sums of each of the plurality of degrees of structure similarity and each of the plurality of degrees of charge similarity;
determining a second molecule whose total degree of similarly is largest among the plurality of kinds of molecules;
acquiring energy of a molecular structure of the target molecule based on an interaction potential of the second molecule; and
determining whether the calculated energy satisfies a certain condition.

8. A stable structure search program that causes at least one computer to execute a process, the process comprising:
acquiring a plurality of degrees of structure similarity between each of a plurality of kinds of molecules and a first molecule included in a target molecule based on each interaction potential of the plurality of kinds of molecules;
acquiring a plurality of degrees of charge similarity between each of the plurality of kinds of molecules and the first molecule;
acquiring a plurality of total degrees of similarity between each of the plurality of kinds of molecules and the first molecule based on sums of each of the plurality of degrees of structure similarity and each of the plurality of degrees of charge similarity;
determining a second molecule whose total degree of similarly is largest among the plurality of kinds of molecules;
acquiring energy of a molecular structure of the target molecule based on an interaction potential of the second molecule; and
determining whether the calculated energy satisfies a certain condition.
